# EUROPEAN PATENT APPLICATION

(11) **EP 4 843 495 A1**
(43) Date of publication of application: **25.11.2026**
(21) Application number: 24917647.0
(22) Date of filing: 16.01.2024
(51) Int. Cl.: A61B 5/1473, A61B 5/1486

(54) **BODY FLUID ANALYTE TEST DEVICE**

(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2024/072438
(87) International publication number: WO 2025/152019

(57) **Abstract**

The invention discloses a body fluid analyte detection device, the transmitter is releasably connected to the bottom case. After the user installs the disposable bottom case onto the skin surface, and then assembles the reusable transmitter onto the bottom case, a complete analyte detection device can be formed. When the user replaces the analyte detection device, they only need to apply force to one side of the bottom case to bend it and make the transmitter and the bottom case separate. Then, they can remove the bottom case from the skin to complete the disassembly. The bottom case is provided with a crease groove, which facilitates the bending of the bottom case. The structure is simple, the installation and disassembly operations are convenient, time-saving and labor-saving, and enhances the user's experience.

## Description

### TECHNICAL FIELD

The invention mainly relates to the field of medical device, and in particular to a body fluid analyte detection device.

### BACKGROUND

The pancreas in the normal human body automatically monitors the glucose content in the human blood and automatically secretes the required insulin/glucagon. Diabetic patients have abnormal pancreas function and fail to normally secrete human insulin. Diabetes mellitus is therefore a metabolic disease caused by abnormal pancreatic function in humans, and diabetes mellitus is a lifelong disease. Current medical technology has not been able to eradicate diabetes and can only control the development and development of diabetes and its complications by stabilizing blood glucose.

Diabetic patients need to detect blood glucose before injecting insulin into the body. Most of the current detection methods allow continuous detection of blood glucose and transmit the blood glucose data in real time to a remote device for viewing by the user, referred to as Continuous Glucose Monitoring (CGM). The method requires the detection device to be attached to the skin surface and the sensor probe carried by it is inserted into the subcutaneous interstitial fluid to perform the detection.

The current body fluid analyte detection devices cannot well separate the transmitter and the bottom case, and the disassembly is inconvenient, which affects the user experience.

Therefore, the existing technology urgently needs a body fluid analyte detection device with convenient disassembly operation.

### BRIEF SUMMARY OF THE INVENTION

This embodiment of the invention discloses a body fluid analyte detection device, the transmitter is releasably connected to the bottom case. After the user installs the disposable bottom case onto the skin surface, and then assembles the reusable transmitter onto the bottom case, a complete analyte detection device can be formed. When the user replaces the analyte detection device, they only need to apply force to one side of the bottom case to bend it and make the transmitter and the bottom case separate. Then, they can remove the bottom case from the skin to complete the disassembly. The bottom case is provided with a crease groove, which facilitates the bending of the bottom case. The structure is simple, the installation and disassembly operations are convenient, time-saving and labor-saving, and enhances the user's experience.

This invention discloses a body fluid analyte detection device, which comprises: a bottom case; a transmitter, configured to establish a communication connection with a remote device; a battery, configured to provide electrical energy for the body fluid analyte detection device; a sensor and a conductive silicone strip installed on the bottom case; the sensor comprising an in vivo part and an in vitro part, the in vivo part is used to penetrate the subcutaneous tissue to detect the parameters of the body fluid analyte information, when the transmitter is assembled onto the bottom case, the in vitro part is electrically connected to the transmitter through the conductive silicone strip to transmit the analyte parameters information; and an adhesive tape, configured to stick the bottom case onto the skin surface; wherein the bottom case is provided with a crease groove, the crease groove is thinner relative to the bottom surface of the bottom case, the crease groove comprises a straight section and a curved section, when separating the bottom case and the transmitter, the bottom case bends and fails along the crease groove.

According to one aspect of the invention, the curved section is located at both ends of the straight section.

According to one aspect of the invention, the curved section and/or the straight section are semi-open structures.

According to one aspect of the invention, the conductive silicone strip is the rectangular structure.

According to one aspect of the invention, the conductive silicone strip comprises conductive areas and insulating areas longitudinally spaced apart.

According to one aspect of the invention, the transmitter comprises a first electrical connection area, the first electrical connection area and the in vitro part are respectively electrically connected to the conductive areas on the conductive silicone strip.

According to one aspect of the invention, the first electrical connection area and the in vitro part are respectively electrically connected to the conductive areas on the adjacent or opposite surfaces of the conductive silicone strip.

According to one aspect of the invention, at least one non-electrical connection structure surface of the conductive silicone strip is covered with insulating material.

According to one aspect of the invention, the bottom case comprises at least one first engagement part, the transmitter comprises at least one second engagement part, when the transmitter is assembled onto the bottom case, the first engagement part is engaged with the second engagement part.

According to one aspect of the invention, when the bottom case fails, the first engagement part decouples from the second engagement part.

According to one aspect of the invention, the first engagement part is located on the curved side wall of the bottom case.

According to one aspect of the invention, the bottom case comprises a battery cavity, the battery is set in the battery cavity.

According to one aspect of the invention, the battery cavity comprises a cavity shell, the cavity shell is used as the shell of the battery.

According to one aspect of the invention, the battery cavity comprises a separable cavity cover.

According to one aspect of the invention, the device also comprises an auxiliary installer, which is configured to install the bottom case onto the skin surface and insert the in vivo part into the subcutaneous tissue.

According to one aspect of the invention, before installation, the bottom case and the auxiliary installer are releasably connected, and the center axis of the bottom case and the auxiliary installer are coincident or not coincident.

According to one aspect of the invention, the failure mode of the bottom case comprises bottom case fracture and bottom case bending deformation.

According to one aspect of the invention, the in vitro part bends relative to the in vivo part.

Compared with the prior art, the technical solution of the invention has the following advantages:
This embodiment of the invention discloses a body fluid analyte detection device, the transmitter is releasably connected to the bottom case. After the user installs the disposable bottom case onto the skin surface, and then assembles the reusable transmitter onto the bottom case, a complete analyte detection device can be formed. When the user replaces the analyte detection device, they only need to apply force to one side of the bottom case to bend it and make the transmitter and the bottom case separate. Then, they can remove the bottom case from the skin to complete the disassembly. The bottom case is provided with a crease groove, which facilitates the bending of the bottom case. The structure is simple, the installation and disassembly operations are convenient, time-saving and labor-saving, and enhances the user's experience.

Further, the crease groove on the bottom case comprises a straight section and a curved section. The combination of the straight section and the curved section crease grooves enables the bottom case to fail while maintaining a certain strength, preventing abnormal failure of the bottom case due to movement or other activities by the user during daily use, which may cause the transmitter to detach prematurely from the bottom case and affect the user's use.

Further, the curved section and/or the straight section is a semi-open structure, that is, the bottom surfaces of the straight section or the curved section is intermittently connected through the bottom case material, and the unconnected parts are completely removed. This can reduce the overall weight of the analyte detection device.

Further, the electrical connection area between the in vivo part and the transmitter is electrically connected on the adjacent surfaces of the conductive silicone strip, that is, the in vivo part is electrically connected to the side edge of the conductive silicone strip. This can reduce the overall thickness of the sensor in vitro part and the conductive silicone strip, and further reduce the overall thickness of the body fluid analyte detection device, which is conducive to the miniaturization design of the detection device.

Further, at least one non-electrical connection surface of the conductive silicone strip is covered with an insulating material, which can prevent the non-electrical connection surface of the conductive silicone strip from being contaminated by iron filings or other conductive dirt, causing adjacent or close conductive areas to short circuit and affecting the detection signal.

Further, the in vitro part is tiled on the bottom case and no additional base is needed to fix the sensor, reducing the number of structural components of the detection device and improving the assembly integration. This is conducive to the miniaturization design of the detection device. When installing the detection device, the process of installing the sensor onto the bottom case is omitted, simplifying the installation steps and facilitating user use.

Further, the cavity shell of the battery cavity can be integrally formed with the shell of battery, that is, the battery cavity itself serves as the shell of battery, and there is no need to assemble a disposable battery or a rechargeable battery into the battery cavity. This simplifies the production process flow, at the same time, since there is no longer a shell of battery, more electrolyte can be set in the battery cavity, improving the energy storage of the battery cavity and extending the service life of the detection device, further, if the energy provided by the electrolyte can meet the service life of the detection device, the volume of the battery cavity can be reduced, which is conducive to the miniaturization design of the detection device.

Further, the battery cavity can also comprise a separable cavity cover for sealing the battery in the battery cavity. In the production process, it is convenient to assemble the independent battery into the battery cavity.

### BRIEF DESCRIPTION OF THE DRAWINGS

FIG. 1a is a block diagram of an auxiliary installer according to one embodiment of the invention;
FIG. 1b is a block diagram of an analyte detection device and an auxiliary installer according to one embodiment of the invention;
FIG. 1c is a schematic view of a piercing structure, a parallel slider, and a bottom case according to one embodiment of the invention;
FIG. 2a is a block diagram of an analyte detection device according to one embodiment of the invention;
FIGS. 2b-2c illustrate exploded configurations of analyte detection devices according to various embodiments of the invention;
FIG. 3 is a block diagram of a transmitter according to one embodiment of the invention;
FIG. 4 is a structural diagram of a bottom case according to one embodiment of the invention;
FIGS. 5 a and 5b are schematic diagrams illustrating the structure of the bottom case before and after failure according to one embodiment of the invention;
FIGS. 5c and 5d illustrate the structure of the bottom case before and after failure of the third engagement part according to one embodiment of the invention;
FIG. 6 is a schematic view of a cross-sectional view X-X' of the battery cavity of FIG. 2b according to one embodiment of the invention;
FIG. 7a illustrates a structure of a conductive silicone strip according to one embodiment of the invention;
FIG. 7b illustrates a structure where one structure surface of the conductive silicone strip is sealed according to one embodiment of the invention;
FIGS. 7c-7f illustrate a configuration, in which a sensor and a conductive silicone strip are assembled to a bottom case, according to one embodiment of the invention;
FIG. 7g illustrates a structure, in which the conductive area of the conductive silicone strip is sealed, according to one embodiment of the invention.

### DETAILED DESCRIPTION

As mentioned earlier, the existing detection devices cannot effectively separate the transmitter and the base case, making disassembly inconvenient and affecting the user experience.

To solve this problem, the invention provides a body fluid analyte detection device. The transmitter is releasably connected to the bottom case. After the user installs the disposable bottom case onto the skin surface, and then assembles the reusable transmitter onto the bottom case, a complete analyte detection device can be formed. When the user replaces the analyte detection device, they only need to apply force to one side of the bottom case to bend it and make the transmitter and the bottom case separate. Then, they can remove the bottom case from the skin to complete the disassembly. The bottom case is provided with a crease groove, which facilitates the bending of the bottom case. The structure is simple, the installation and disassembly operations are convenient, time-saving and labor-saving, and enhances the user's experience.

Various exemplary embodiments of the invention will now be described in detail with reference to the accompanying drawings. It is to be understood that the relative arrangement of the components and steps, numerical expressions and numerical values set forth in these examples should not be construed as limiting the scope of the invention unless specifically stated otherwise.

Further, it is to be understood that the dimensions of various components illustrated in the figures are not necessarily drawn to actual scale for ease of description, e.g., the thickness, width, length, or distance of some elements may be exaggerated relative to other structures.

The following description of exemplary embodiment(s) is merely illustrative and is not to be taken in any sense as a limitation on the invention, its application or uses. Techniques, methods, and apparatus known to those of ordinary skill in the relevant art may not be discussed in detail herein, but where applicable, such techniques, methods, and apparatus should be considered part of this specification.

It should be noted that like reference numerals and letters denote like items in the following figures, and thus, once an item is defined or illustrated in one of the figures, further discussion thereof will not be necessary in the subsequent description of the figures.

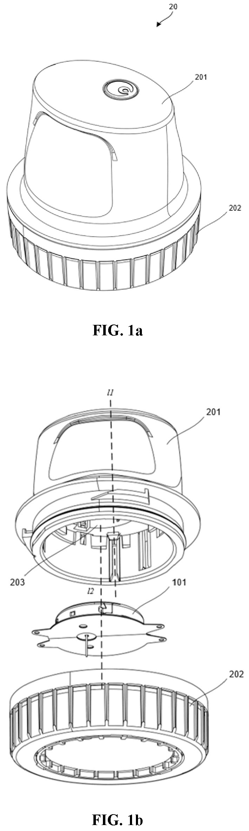

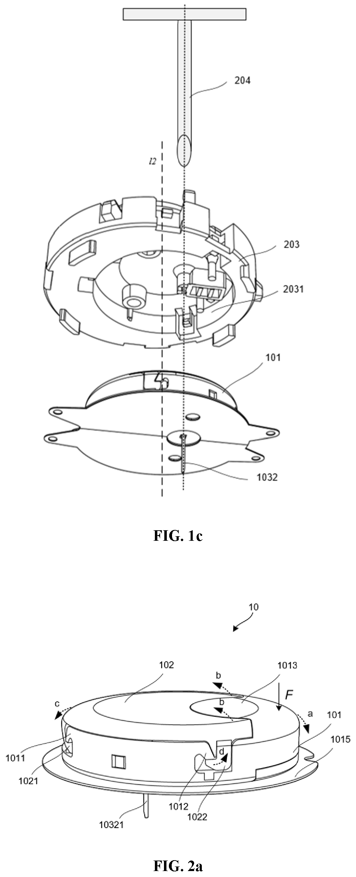

FIG. 1a is a block diagram of an auxiliary installer according to one embodiment of the invention. FIG. 1b is a block diagram of an analyte detection device and an auxiliary installer according to one embodiment of the invention. FIG. 1c is a schematic view of a piercing structure according to one embodiment of the invention, in which parallel sliders are coupled to a bottom case.

In one embodiment of the invention, the auxiliary installer 20 comprises a housing 201 and a protective cover 202, the analyte detection device 10 is positioned within the housing 201, and the analyte detection device 10 is mounted to the skin surface by a user unscrewing the protective cover 202 from the housing 201 and pressing the housing 201 against the skin surface when required for mounting. In an embodiment of the invention, the sensor, antenna, circuit board, and battery are all disposed within the shell of the analyte detection device 10 as a unitary structure.

In another embodiment of the invention, only the bottom case 101 of the analyte detection device 10 is contained within the housing 201 of the auxiliary installer 20. When required for mounting, the bottom case 101 is mounted to the skin surface by pressing the housing 201 against the skin surface, and then the transmitter 102 is assembled to the bottom case 101. In the embodiment of the invention, the antenna and the circuit board are provided in the transmitter 102, and the sensor and the battery are provided on the bottom case 101, forming a split structure. In an embodiment of the invention, the bottom case 101 is disposable and the transmitter 102 is reusable.

In some embodiments of the invention, prior to installation, the bottom case 101 is releasably coupled to the auxiliary installer 20. The bottom case 101 is circular or approximately circular having a central axis *l2* and the auxiliary installer has a central axis *l1.* In some embodiments of the invention, the central axis *l1* coincides with *l2.* In other embodiments of the invention, the central axes *l1* and *l2* do not coincide.

In some embodiments of the invention, a parallel slider 203 is also comprised in the auxiliary installer 20, before installation, the bottom case 101 is releasably connected with the parallel sliders 203, the parallel slider 203 is provided with a recess 2031 for receiving the bottom case 101, which has a shape corresponding to the shape of the bottom case 101, is circular or approximately circular, and shares a central axis with the bottom case 101. The size of the recess 2031 on the parallel slider 203 is adapted to vary regardless of the size of the bottom case 101 during the iteration of the version of the analyte detection device 10.

Referring to FIG. lc, in some embodiments of the invention, with iterations of the analyte detection device 10, the auxiliary installer 20 can be adapted to different versions of the analyte detection device 10 to save on development of the auxiliary installer 20, the analyte detection device 10, production costs and cycle time, and different versions of the analyte detection device 10 often have different dimensions, based on which some adaptations to the structure of the analyte detection device 10 are required.

For example, in some embodiments of the invention, when the bottom case 101 of some larger-sized analyte detection devices 10 is fixed on the parallel slider 203 through the recess 2031, the central axis *l2* of the bottom case 101 coincides with the central axis *l1* of the auxiliary installer 20 (with the central axis of the parallel slider 203), the recess 2031 is now also coaxial with the auxiliary installer 20, the bottom case 101, and a piercing structure 204, such as a steel needle, is coaxial with the sensor 1032 to accommodate the sensor 1032, while when the auxiliary installer 20 changes to fit a smaller size of the bottom case 101, if the location of the piercing structure 204 on the auxiliary installer 20 remains the same, i.e. The position of the sensor 1032 remains unchanged, the central axis *l2* of the bottom case 101 will then be offset from the central axis *l1* of the auxiliary installer 20 and close to the piercing structure 204, the two will no longer coincide, structural modification of the parallel slider 203 is required at this point, for example, when the recess 2031 is integrally adjacent to the piercing structure 204 and its circumference is adapted to decrease with the bottom case 101 so that the recess 2031 can receive the bottom case 101 while the piercing structure 204 can still pass through the bottom case 101 and receive the sensor 1032, the central axis of the recess 2031 is offset from the central axis *l1* of the auxiliary installer 20 and is in an eccentric position with respect to the parallel slider 203. Alternatively, in other embodiments of the invention, if the center axis of the bottom case 101 and the auxiliary installer 20 are kept coincident, i.e., the position of the recess 2031 is constant, the piercing structure 204 needs to be positioned closer to the center axis *l2* of the bottom case 101. Since the piercing structure 204 is fixed inside the housing 201, the internal housing structure of the housing 201 needs to be modified. For the auxiliary installer 20, it is easier to change the position of the recess 2031 of the parallel slider 203, less expensive to design, manufacture, and less periodic than changing the position of the piercing structure 204. Therefore, in the preferred embodiment of the invention, it is possible to adaptively change the size of the recess 2031 and its position on the parallel slider 203 in order to accommodate the smaller size of the analyte detection device 10.

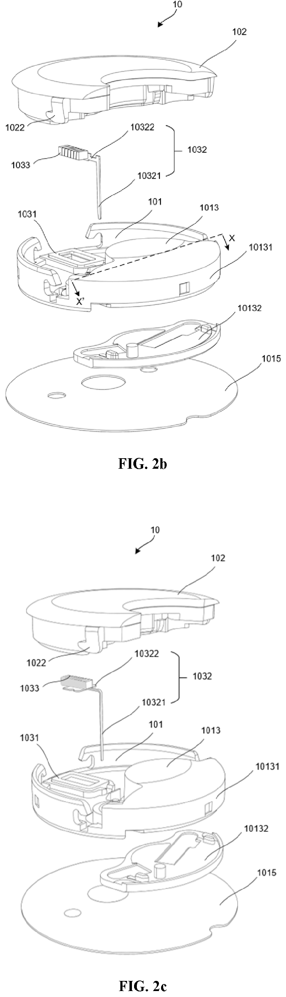

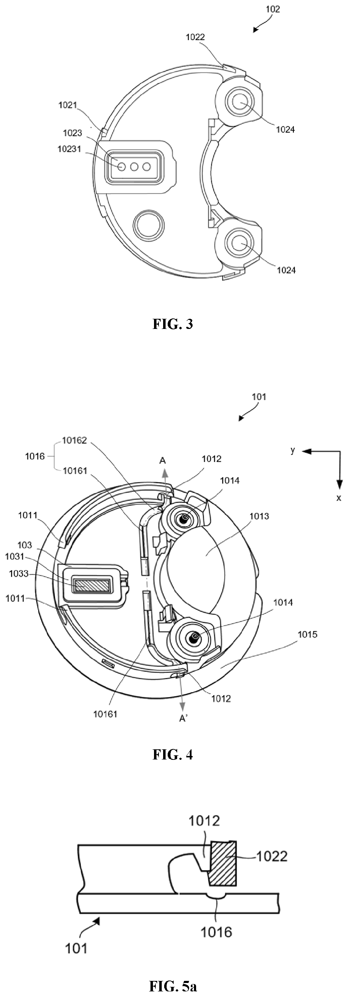

FIG. 2a is a block diagram of an analyte detection device according to one embodiment of the invention. Figs. 2b-2c illustrate exploded configurations of analyte detection devices according to various embodiments of the invention. FIG. 3 is a block diagram of a transmitter according to one embodiment of the invention. FIG. 4 illustrates a structure of a bottom case according to one embodiment of the invention.

In some embodiments of the invention, the analyte detection device 10 comprises a bottom case 101, a transmitter 102, and a sensor module 103.

In some embodiments of the invention, bottom case 101 is releasably coupled to transmitter 102. The bottom case 101 comprises at least one first engagement part 1011, and the transmitter 102 is provided with at least one second engagement part 1021 at a corresponding position, and the first engagement part 1011 and the second engagement part 1021 can be engaged with each other, so that the transmitter 102 can be fixed on the bottom case 101.

In some embodiments of the invention, the first engagement part 1011 and the second engagement part 1021 may be engaged with a hook and a slot, a hook and a hole, a hook and a hook, or the like, without limitation.

In some embodiments of the invention, the first engagement part 1011 and the second engagement part 1021 are engaged with each other so that the transmitter 102 and the bottom case 101 are securely connected to each other, and the transmitter 102 is not easily detached from the bottom case 101.

In some embodiments of the invention, since the analyte detection device 10 is circular or nearly circular, the sidewalls of the bottom case 101 and the transmitter 102 are curved or rounded, which are compatible in shape and size.

In some embodiments of the invention, at least two first engagement parts 1011 are symmetrically disposed on the curved side wall of the bottom case 101 to constrain the transmitter 102, and correspondingly, at least two second engagement parts 1021 are disposed at corresponding positions on the curved side wall of the transmitter 102.

In one embodiment of the invention, at least two third engagement parts 1012 and at least two fourth engagement parts 1022 may be provided on the bottom case 101 and the transmitter 102, respectively, in order to secure the transmitter 102 to the bottom case 101, the fourth engagement parts 1022 having positions, shapes and numbers corresponding to the third engagement parts 1012.

In some embodiments of the invention, since the bottom case 101 has a circular or near-circular shape, the third engagement parts 1012 are also distributed on the curved side wall of the bottom case 101.

In some embodiments of the invention, the third engagement parts 1012 are symmetrically distributed on the side wall of the bottom case 101.

In one embodiment of the invention, taking the bottom case 101 shown in FIG. 2a for reference, each of the first engagement part 1011 and the third engagement part 1012 is located on a side wall of the bottom case 101, the first engagement part 1011 is located at a left end position of a side wall of the bottom case 101, the third engagement part 1012 is located at an intermediate position of the side wall of the bottom case 101, the position is determined by the specific shape of the transmitter 102, so as to be able to fix both ends of the transmitter 102 on the bottom case 101, respectively, thereby maintaining a tight snap connection of the transmitter 102 to the bottom case 101, accordingly, it is contemplated that the positions, shapes, and numbers of the first engagement part 1011 (the second engagement part 1021) and the third engagement part 1012 (the fourth engagement part 1022) may be adapted as the shape of the transmitter 102 is changed, and that the positions, shapes, and numbers of the first engagement part 1011 (the second engagement part 1021) and the third engagement part 1012 (the fourth engagement part 1022) may be changed regardless of the changes in the positions, shapes, and numbers of the first engagement part 1011 (the second engagement part 1021) and the third engagement part 1012 (the fourth engagement part 1022) are comprised within the scope of the invention.

In some embodiments of the invention, the third engagement part 1012 is disposed on a curved side wall of the bottom case 101, as shown in FIG. 3. In other embodiments of the invention, the third engagement part 1012 is provided on the bottom surface of the bottom case 101. In still other embodiments of the invention, the third engagement part 1012 may be provided on both the curved side wall and the bottom surface of the bottom case 1012, respectively, without limitation.

In the preferred embodiment of the invention, the bottom case 101 is provided with a first engagement part 1011 and a third engagement part 1012, and the transmitter 102 is provided with a second engagement part 1021 and a fourth engagement part 1022, the positions, shapes, and numbers of the second engagement part 1021 and the fourth engagement part 1022 are respectively adapted to the first engagement part 1011 and the third engagement part 1012.

In some embodiments of the invention, the transmitter 102 can be stably fixed to the bottom case 101 by engaging the first engaging part 1011 and the third engaging part 1012 with the second engaging part 1021 and the fourth engaging part 1022, respectively. Since the transmitter 102 is reusable, when a user replaces a new analyte detection device 10, the transmitter 102 needs to be removed from the bottom case 101, which allows the bottom case 101 to flex and fail. When the bottom case 101 is bent to fail, the engagement of the first engagement part 1011 with the second engagement part 1021 or/and the engagement of the third engagement part 1012 with the fourth engagement part 1022 are decoupled, thereby separating the transmitter 102 and the bottom case 101. See below for specific details.

In some embodiments of the invention, in order for the bottom case 101 to fail in bending, the bottom case 101 is made of a flexible material, such as PE or PP plastic. The bottom case 101 needs to be adhered to the skin surface of the user during use, as arm, on belly, while the user's skin surface during daily activities uninterrupted peristalsis or bending, the too flexible bottom case 101 is also bent, which causes the first engagement part 1011 to be engaged with the second engagement part 1021 or/and the third engagement part 1012 to be engaged with the fourth engagement part 1022 to be uncoupled with a certain probability, so that the transmitter 102 and the bottom case 101 are loosened, on the basis of which the bottom case 101 cannot be too flexible.

In some embodiments of the invention, a crease groove 1016 is provided on the bottom case 101 to maintain some rigidity of the bottom case 101 while also allowing a user to flex the bottom case 101 to decouple the engagement parts after failure of the bottom case 101. The bottom face thickness at crease groove 1016 is slightly thinner than the other bottom faces, for example, when the bottom case 101 has a thickness of 0.5 to 1.0mm, the bottom surface at the crease groove 1016 is relatively reduced in thickness by 0.01-0.7 mm, or half-strand of the bottom surface at the crease groove 1016 (no bottom case material is provided at the strand, where the thickness of the bottom surface is 0mm), that is, the bottom surface of both sides of the crease groove 1016 is intermittently connected by the material of the bottom case 101, or the bottom surface of the bottom case 101 is not provided with the crease groove 1016, but the bottom surface of the bottom case 101 is provided only in a half-strand empty structure, and the bottom surface of both sides of the bottom case is connected to facilitate bending of the bottom case 101 by the user.

Referring to FIG. 4, in some embodiments of the invention, with reference to the x-y coordinates, the x-y coordinates are planar coordinates of the bottom case 101, and the crease groove 1016 is a linear groove parallel to the x-axis. When the bottom case 101 is bent, the bottom of the bottom case 101 on both sides of the crease groove 1016 is warped in a direction perpendicular to the x-y coordinate plane.

In other embodiments of the invention, the crease groove 1016 is formed by combining a straight section 10161 and a curved section 10162 to form the A-A' trajectory as shown in FIG. 4, the straight section 10161 being parallel to the x-axis, and the curved section 10162 being disposed at both ends of the straight section 10161. The linear portion 10161 groove may facilitate bending of the bottom case 101, the curved section 10162 may also facilitate bending of the bottom case 101, except that the curved section 10162 has a portion at an angle to the x-axis, which may increase the bending strength of the crease groove 1016, increase the design redundancy of stiffness to the bottom case 101, make the crease groove 1016 less flexible during daily use, and increase the stability of the connection of the transmitter 102 to the bottom case 101.

In some embodiments of the invention, crease groove 1016 may also be used in conjunction with third engagement part 1012 and fourth engagement part 1022 to complete engagement and disengagement of bottom case 101 with transmitter 102, as described below.

In some embodiments of the invention, the bottom surface at the straight section 10161 or at the curved section 10162 may also be partially empty, i.e., the bottom surface at both sides of the straight section 10161 or at the curved section 10162 is intermittently connected by the material of the bottom case 101, and the unconnected portions are completely removed, thereby reducing the overall weight of the analyte detection device 10.

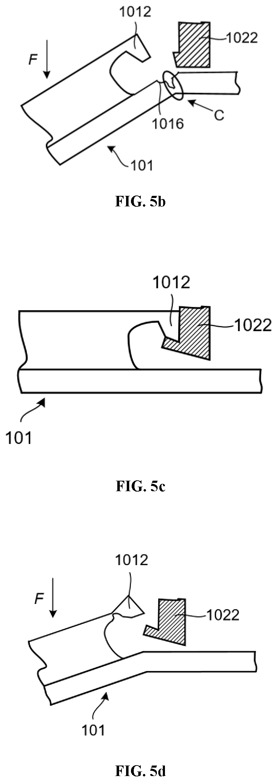

FIGS. 5a and 5b are schematic diagrams illustrating the structure of the bottom case before and after failure according to an embodiment of the invention. FIGS. 5c and 5d are schematic views illustrating the structure of the bottom case before and after failure of the third engagement part according to an embodiment of the invention.

In some embodiments of the invention, whether crease groove 1016 is a linear groove or a linear, curved combination groove, both ends of crease groove 1016 correspond to third engagement part 1012 and fourth engagement part 1022.

In some embodiments of the invention, the bottom case 101 comprises a fixing part and a force application part, and when the bottom case 101 is bent to expect separation of the transmitter 102 by a user, the fixing part is fixed with a finger, and the force application part is applied with a pressure F, so that the bottom case 101 can be bent along the crease groove 1016 or the bending fails, and the third engagement part 1012 and the fourth engagement part 1022 are decoupled. Here, the fixing part and the force application part are an opposite concept, which will be described in detail below.

In some embodiments of the invention, failure is a conventional concept in the field of engineering materials. After failure, the material loses its original function and the failure site cannot be restored again. Since the third engagement part 1012 is part of the bottom case 101, failure of the bottom case 101 comprises failure of a bottom surface, a side wall of the bottom case 101, or the third engagement part 1012. Thus, the failure mode of the bottom case 101 comprises breaking of the bottom case 101, bending of the bottom case 101, and may comprise breaking of the third engagement part 1012. Clearly, after the bottom case 101 fails, the bottom case 101 loses the function and function of the snap-fit transmitter 102.

In some embodiments of the invention, the manner of fixing the fixing part comprises clamping, supporting, etc., which is not particularly limited herein, as long as the condition for fixing the fixing part can be satisfied. Specifically, the crease groove 1016 divides the bottom case into two sides, in which one side functions as a fixing part and the other side functions as a force application part, and the fixing part and the force application part are interchangeable with each other.

Referring to FIGS. 5a and 5b, in some embodiments of the invention, the process of separating the bottom case 101 and the transmitter 102 is as follows: the fixing part is fixed with a finger, the second finger is applied with a force F in one direction to the force application part to bend or bend the bottom case 101, and the fourth engagement part 1022 is decoupled from the engagement of the third engagement part 1012 to separate the transmitter 102 from the bottom case 101. Obviously, the bottom case 101 is bent or curved along the crease groove 1016. The cooperation of the crease groove with the third engagement part and fourth engagement part enables better separation of the transmitter and the bottom case.

Referring to FIGS. 5c and 5d, in some embodiments of the invention, the process of separating the bottom case 101 and the transmitter 102 is as follows: fixing the fixing part with a finger, applying a force F to the force application part with another finger in one direction to disable the third engagement part 1012, thereby separating the third engagement part 1012 from the fourth engagement part 1022 to separate the transmitter 102 from the bottom case 101.

In some embodiments of the invention, the bottom case 101 is also provided with a battery cavity 1013, in which a battery is mounted to provide electrical power to the analyte detection device 10.

With combined reference to FIG. 2a, in some embodiments of the invention, the battery cavity 1013 serves as the force application part and the transmitter 102 serves as the securing portion. Upon separating the transmitter 102 and the bottom case 101, a user's finger holds the transmitter 102, the other finger exerting a force F on the battery cavity 1013, bending the battery cavity 1013 in a curve direction, the fourth engagement part 1022 has a movement tendency in a d-curve direction with respect to the battery cavity 1013, as the force continues to be applied to the battery cavity 1013, the bottom case 101 bending deformation, the bottom case 101 no longer abuts against the fourth engagement part 1022, the transmitter 102 moves in a b-curve direction and a c-curve direction relative to the battery cavity 1013, until the fourth catch 1022 is decoupled from the third catch 1012, at this time, since the first engagement part 1011 and the second engagement part 1021 are not completely decoupled, the transmitter 102 is in a semi-coupled state with the bottom case 101, so that the transmitter 102 can be prevented from falling freely during the disassembly process, and the user needs to take the transmitter 102 with a finger to release the engagement state of the first engagement part 1011 and the second engagement part 1021, thereby completing the disassembly of the transmitter 102, and the operation is convenient.

As described above, the force application part and the fixing part are opposite, and in other embodiments of the invention, the transmitter 102 is the force application part and the battery cavity 1013 is the fixing part, and the disassembly process is consistent.

In some embodiments of the invention, the battery cavity 1013 further comprises a detachable cavity cover 10132. The detachable cavity cover 10132 is used to fit the battery or electrolyte into the battery cavity 1013 during production. The cavity cover 10132 is secured to the battery cavity 1013 and sealed after production to prevent contaminants such as water droplets from entering the battery cavity 1013. After the cavity cover 10132 is sealed, the adhesive tape 1015 is adhesively secured to the bottom case 101, and the cavity cover 10132 is covered with the adhesive tape 1015.

In some embodiments of the invention, the cavity cover 10132 is attached to the cavity shell 10131 by gluing, snapping, welding, etc., to be secured to the battery cavity 1013. It will be appreciated that the cavity cover 10132 may be releasably attached to the cavity shell 10131 prior to manufacture, or in a separate state, and the cavity cover 10132 may be fixedly attached to the cavity shell 10131 after installation of the battery, so that the cavity cover 10132 may be completely sealed from the cavity shell 10131, so that the cavity cover 10132 cannot be removed again, so that the cavity cover 10132 is prevented from loosening and external dirt may enter the battery cavity 1013.

In some embodiments of the invention, the battery is a self-contained coin battery and the battery is received in the battery cavity 1013.

In some embodiments of the invention, the positive electrode and the negative electrode of the battery are electrically connected to the elastic conductor 1014 respectively, the other end of the elastic conductor 1014 is electrically connected to the second electrical connection area 1024 of the transmitter 102, and the elastic conductor 1014 acts as a conductive carrier, so that the battery can supply the electric energy to the transmitter 102. The second electrical connection area 1024 is compatible with the resilient electrical conductors 1014 in a consistent number.

In some embodiments of the invention, when the transmitter 102 is assembled to the bottom case 101, the second electrical connection area 1024 contacts and compresses the elastic conductor 1014. The elastic conductor 1014 is in a compressed state to be in closer contact with the second electrical connection area 1024. Second, when the transmitter 102 is disassembled, the elastic force of the elastic conductor 1014 also assists the separation of the transmitter 102 from the bottom case 101 after the third engagement part 1012 is decoupled from the fourth engagement part 1022.

In some embodiments of the invention, the resilient electrical conductor 1014 may be a conductive spring, a conductive rubber, or the like.

In some embodiments of the invention, the second electrical connection area 1024 is a metal contact.

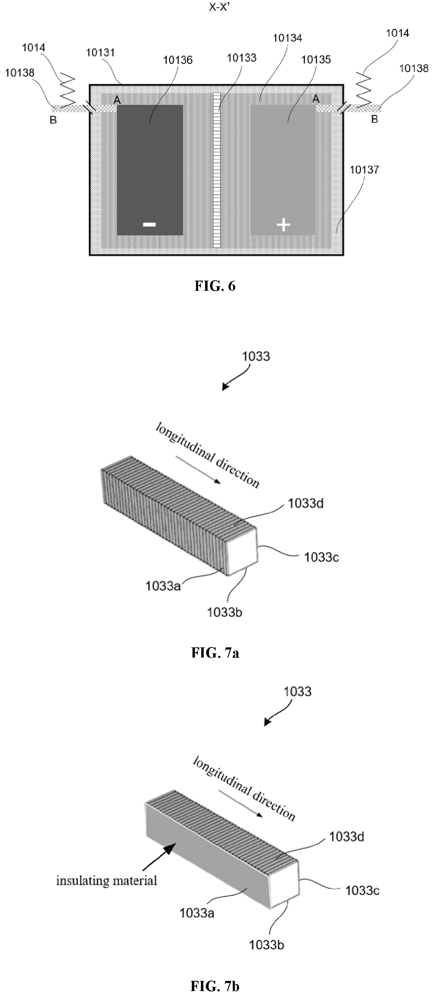

In other embodiments of the invention, the battery cavity 1013 itself acts as a battery to provide electrical power to the analyte detection device 10, as shown in more detail in FIG. 6.

FIG. 6 is a schematic view of a cross-sectional view X-X 'of the battery cavity of FIG. 2b, according to an embodiment of the invention.

In some embodiments of the invention, the battery cavity 1013 comprises a cavity shell 10131 that is integrally formed with the bottom case 101 to allow for more miniaturization of the analyte detection device 10. In some embodiments of the invention, battery cavity 1013 comprises cavity shell 10131, diaphragm 10133, electrolyte 10134, anode plate 10135, cathode plate 10136, electrolyte separator 10137, and conductive strip 10138. Cavity shell 10131 is used to house and hold the structure described above, with anode plate 10135 and cathode plate 10136 immersed in electrolyte 10134, separated by diaphragm 10133. According to the above structure, the battery cavity 1013 itself can be used as a complete battery to provide electrical power to the analyte detection device 10, and the housing of the battery cavity shell be used as a battery housing, eliminating the need for a separate battery housing, and the battery cavity 1013 can be more miniaturized while still containing more electrolyte 10134, storing more electrical power, and extending the life of the analyte detection device 10. In some embodiments of the invention, diaphragm 10133, anode plate 10135, and cathode plate 10136 are in a wound configuration, and diaphragm 10133 is positioned between anode plate 10135 and cathode plate 10136.

In some embodiments of the invention, diaphragm 10133, anode plate 10135, and cathode plate 10136 are in a stacked planar configuration, and diaphragm 10133, anode plate 10135, and cathode plate 10136 are spaced apart from each other.

In some embodiments of the invention, the electrolyte 10134 is a lithium salt, such as one of lithium perchlorate (LiCIO₄), lithium hexafluorophosphate (LiPF₆), lithium tetrafluoroborate (LiBF₄). The solvent is one of ethylene carbonate, propylene carbonate, diethyl carbonate, dimethyl carbonate, ethyl methyl carbonate, phosphorus pentafluoride, hydrofluoric acid, diethyl ether, ethylene carbonate, propylene carbonate, diethyl carbonate. In a preferred embodiment of the invention, the solvent is selected from an organic solvent such as one of diethyl ether, ethylene carbonate, propylene carbonate, diethyl carbonate.

In some embodiments of the present disclosure, the main material of anode plate 10135 is manganese dioxide and is made by the following fabrication process:
① Screening of electrolytic manganese dioxide, conductive agent, binder, which can be done by a screen or a gas flow classifier, selecting electrolytic manganese dioxide particles having a particle size of less than 200 um, placing in a quartz boat, and heat treating in a sintering furnace at a temperature of 200 °C for 4 h. The purpose of this step is to cause the electrolytic manganese dioxide to lose part of the bound water, the X-ray diffraction peaks to shift, the interplanar spacing to decrease, and the Mn-0 bonding to increase, resulting in an increase in the discharge capacity of the electrolytic manganese dioxide.
② After cooling down the electrolytic manganese dioxide in step ① to 60 °C or less, 9g of electrolytic manganese dioxide, 0.5g of a conductive agent having a particle size of less than 200 um, and 0.5g of a binder having a particle size of less than 200um were separately weighed with an electronic balance, placed in a grinding dish, thoroughly stirred and mixed, followed by hand or electric grinding to obtain 10g of a ground mixture, and the ground mixture was allowed to pass through a 300 mesh (particle size of 48 um) screen. The purpose of this step is to ensure the homogeneity of the mixture and to avoid non-uniform dispersion of the conductive agent and the additives.

In other embodiments of the invention, the electrolytic manganese dioxide, the conductive agent, and the binder are not limited to the above-mentioned mass proportions, and the mass proportions thereof may be formulated in a ratio of 80% to 96%, 2% to 10%, and 2% to 10%, respectively.

In preferred embodiments of the invention, the conductive agent may be one or more of conductive carbon black, graphite, super p, or carbon nanotubes.

In a preferred embodiment of the invention, the binder may be one or more of PVDF (poly-vinylidene fluoride), polytetrafluoroethylene, sodium polyacrylate.
③ The ground mixture was placed in a vacuum oven and heated to 65 °C for 5 h to dry moisture that may be present in the mixture to ensure that the sample was dry to obtain a positive electrode mixture.
④ 10g of NMP (N-methyl-pyrrolidone) solvent was added dropwise in a dried glass bottle, and then the positive electrode mixture was slowly added into the glass bottle, and stirred with a magnetic stirrer for 3 h, ensuring uniform mixture, to obtain a anode plate with a solid content of 50%. The purpose of this step is to ensure uniform dispersion of the components in the anode plate, and the solid content has a certain relationship with the viscosity of the anode plate, and the anode plate with 50% solid content has a better viscosity, a better film-forming effect after coating on a substrate, and can reduce the phenomenon of dusting or cracking.
⑤ The anode plate was coated on the surface of the substrate using a flat coater to obtain a conductive layer, and then the conductive layer and the substrate were baked in a vacuum oven and heated to 110 °C for 12 h to ensure complete drying of the moisture.

In a preferred embodiment of the invention, the base material is one of aluminum foil or nickel foam mesh with a thickness of 12-18 um.

In a more preferred embodiment of the invention, the base material is aluminum foil with a thickness of 15 um.
⑥ The overall thickness of the conductive layer and the substrate can be reduced to 180-220um by rolling the conductive layer and the substrate using an electric vertical rolling press to obtain a finished anode plate. By adjusting the operating parameters of the coater and roller compactor, the thickness of the anode plate can be controlled to ensure that the anode plate has a relatively perfect conductive network at a relatively high compaction density, so as to accommodate the operating requirements of high current pulse discharges.

In present embodiments, cathode plate 10136 is primarily a lithium-based material.

In other embodiments of the invention, anode plate 10135 may be a lithium-containing compound such as lithium manganate, lithium cobaltate, lithium iron phosphate, and corresponding cathode plate 10136 may be graphite.

In embodiments of the invention, the diaphragm 10133 is PE (polyethylene) or PP (polypropylene), and may be a single layer of PE or PP, or may be a 3 layer of PE or PP.

In some embodiments of the invention, one A-end of the conductive strip 10138 is fixedly connected to the anode plate 10135 or the cathode plate 10136, and the other B-end of the conductive strip 10138 is electrically connected to the elastic current conductor 1014 through the electrolyte insulation layer 10137 and the cavity shell 10131. In a preferred embodiment of the invention, A-end is fixedly connected to anode plate 10135 or cathode plate 10136 by solder or tin paste.

In an embodiment of the invention, the material of the conductive strip 10138 connected to the anode plate 10135 is aluminum, and the material of the conductive strip 10138 connected to the cathode plate 10136 is nickel or copper plated nickel.

In some embodiments of the invention, cavity shell 10131 is typically made of a plastic material, such as PE, PP, or PC, which is easily eroded by electrolyte 10134, so that it may be necessary to provide an electrolyte insulation layer 10137 inside cavity shell 10131.

In some embodiments of the invention, the electrolyte insulation layer 10137 is TPE (thermoplastic elastomer) or PET (polyethylene terephthalate). The TPE is a thermoplastic elastomer material with high processability. The PET material itself acts as a reservoir for the electrolyte and effectively insulates corrosion of the cavity shell by the electrolyte.

In embodiments of the invention, the electrolyte insulation layer 10137 may be a thin film applied to the inside of the cavity shell 10131 by deposition or solution, or may be a separate closed housing.

In a preferred embodiment of the invention, the electrolyte insulation layer 10137 is a thin film of 300-500um thickness. The thickness of the electrolyte insulation layer 10137 is too small to wet out and soften the membrane with the electrolyte, which can lead to aging of the membrane over a long period of time, and the thickness is too large to take up space inside the chamber. In a more preferred embodiment of the invention, the thickness of the electrolyte insulation layer 10137 is 400um.

In some embodiments of the invention, if the cavity shell 10131 is made of a material resistant to electrolyte 10134 corrosion, such as PFA (polytetrafluoroethylene) or FEP (fluoroethylene propylene), the electrolyte insulation layer 10137 may be omitted to increase the volume of electrolyte 10134 and increase the energy storage of the battery.

In some embodiments of the invention, a sensor module is further provided on the bottom case 101, the sensor module comprises an elastic sealing ring 1031, a sensor 1032 and a conductive silicone strip 1033, the sensor 1032 comprises an in vivo part 10321 and an in vitro part 10322.

In some embodiments of the invention, elastic sealing ring 1031 is a ring-shaped structural member, and in vitro part 10322 and conductive silicone strip 1033 are located in an inner ring of elastic sealing ring 1031. When the transmitter 102 is assembled onto the bottom case 101, with reference to FIG. 2b, a lower surface of the elastic sealing ring 1031 contacts a bottom surface of the bottom case 101, the upper surface contacts the shell of the transmitter 102 and forms a completely closed space inside the elastic sealing ring 1031, and the in vitro part 10322 and the conductive silicone strip 1033 are in the closed space, and the closed space of the elastic sealing ring 1031 prevents the ingress of dirt such as water droplets, metal debris, blood, and the like, and contaminates the in vitro part 10322 and the conductive silicone strip 1033, thereby affecting the detection signal.

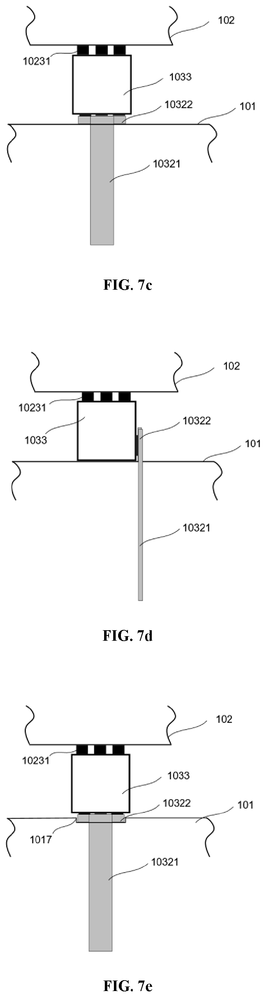

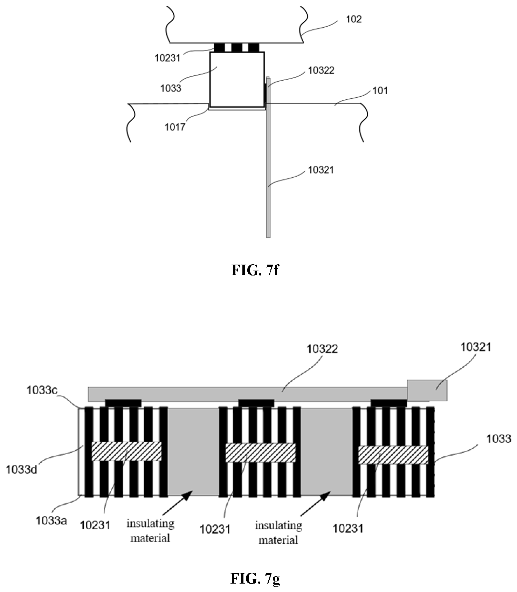

FIG. 7a illustrates a structure of a conductive silicone strip according to an embodiment of the invention. FIG. 7b illustrates a structure of a conductive silicone strip with one structure surface sealed, according to an embodiment of the invention. FIGS. 7c-7f illustrate a configuration of a sensor and a conductive silicone strip mounted to a bottom case, according to an embodiment of the invention. FIG. 7g illustrates a structure, in which a conductive area of a conductive silicone strip is sealed, according to an embodiment of the invention.

Referring to FIG.7a, in an embodiment of the invention, the conductive silicone strip 1033 is a three-dimensional structure having a plurality of structured surfaces, such as a rectangular parallelepiped structure. The conductive silicone strip 1033 has spaced apart conductive areas and insulating areas along a longitudinal length thereof, the conductive areas and the insulating areas extending through a transverse direction of the conductive silicone strip 1033, where the transverse direction is perpendicular to the longitudinal direction. Since both the conductive areas and the insulating areas extend through the lateral direction of the conductive silicone strip 1033, both the conductive areas and the insulating areas are present on the four side surfaces 1033a, 1033b, 1033c, 1033d of the conductive silicone strip 1033, it is understood that the corresponding conductive areas on the four side surfaces 1033a, 1033b, 1033c, 1033d are electrically consecutive.

In some embodiments of the invention, the conductive areas and the insulating areas are spaced apart, the insulating areas can separate two adjacent conductive areas, and the insulating areas have good insulating properties, and can prevent crosstalk of electrical signals on the two adjacent conductive areas, and ensure stability of the detection signal.

In some embodiments of the invention, a conductive silicone strip 1033 is used to electrically connect the sensor 1032 and the transmitter 102, in particular, a first electrical connection area 1023 is comprised on the transmitter, the first electrical connection area 1023 comprises at least two metal contacts 10231, at least two PADs (not shown in the figures) are provided on the in vitro part 10322 of the sensor 1032, respective metal contacts 10231 are in contact with different conductive areas on a single surface of the conductive silicone strip 1033, at the same time, the conductive areas contact the PADs on the same or adjacent or opposite structure surface, thereby electrically connecting the PADs to the metal contacts 10231. The sensing signal of the sensor 1032 can be transmitted to the transmitter 102 through the conductive silicone strip 1033. The transmitter 102 can also transmit the control signal to the sensor 1032 through the conductive silicone strip 1033.

In some embodiments of the invention, the number of metal contacts 10231 is the same as the number of PADs.

In some embodiments of the invention, metal contact 10231 is electrically connected to in vitro part 10322 through opposing surfaces of conductive silicone strip 1033, i.e., 1033a, 1033c or 1033b, 1033d, to form a laminate structure of in vitro part 10322-conductive silicone strip 1033-metal contact 10231, with in vitro part 10322 tiling in a parallel configuration on the bottom surface of bottom case 101, as shown in FIG. 7c.

In some embodiments of the invention, the metal contact 10231 and the in vitro part 10322 are electrically connected through adjacent surfaces of the conductive silicone strip 1033, such that the in vitro part 10322 is electrically connected to a side surface 1033c of the conductive silicone strip 1033, and the metal contact 10231 is electrically connected to an upper surface 1033d, such that the in vitro part 10322 is tiled the bottom surface of the bottom case 101 in a vertical position, as shown in FIG. 7d.

The above embodiments are merely illustrative, and other adjacent surfaces may also perform electrical connection functions. Compared to the stacked structure of the in vitro part 10322-conductive silicone strip 1033-metal contact 10231, making electrical connection to adjacent surfaces may reduce the overall thickness, facilitating a miniaturized design of the analyte detection device 10.

Referring to FIGS. 7e and 7f, in some embodiments of the invention, to further reduce the overall thickness of the analyte detection device 10, a shrinkage pool 1017 may also be provided on the bottom case 101, and an in vitro part 10322 or a conductive silicone strip 1033 may be placed in the shrinkage pool 1017. Placing the in vitro part 10322 or the conductive silicone strip 1033 in the shrinkage pool 1017 may improve assembly stability of the sensor 1032 or the conductive silicone strip 1033 by securing the in vitro part 10322 or the conductive silicone strip 1033 in an interference fit with the shrinkage pool 1017.

In some embodiments of the invention, with reference to the orientation shown in FIG. 2b, in vitro part 10322 is electrically connected to side surface 1033c of conductive silicone strip 1033, and first electrical connection area 1023 of transmitter 102 is electrically connected to upper surface 1033d of conductive silicone strip 1033.

Referring to FIG.s 2a, 7b and 7d in combination, in some embodiments of the invention, a conductive area is also present on the unused surface, the conductive area is contiguous with the conductive area on the used structure. If the conductive area is shorted by dirt, it will also cause the conductive area on the used structure to be shorted and affect the detection signal stability. Therefore, it is contemplated that the conductive area on the unused structure may be sealed to prevent the conductive area from being shorted by dirt. For example, referring to FIG. 7b, in some embodiments of the invention, the in vitro part 10322 is electrically connected to the side surface 1033c of the conductive silicone strip 1033, and the first electrical connection area 1023 of the transmitter 102 is electrically connected to the upper surface 1033d of the conductive silicone strip 1033. When the side surface 1033a and the lower surface 1033b are not in use, an insulating material may be applied to or adhered to the side surface 1033a and/or the lower surface 1033b to prevent the conductive areas on the two surfaces from being shorted by dirt. In the present embodiment, only 1033a is easily contaminated, so it is also possible to coat or stick only 1033a, avoiding waste of insulating material while preventing the conductive area from being shorted by dirt.

Referring to FIG.s 2c, 7b and 7e in combination, in other embodiments of the invention, the in vitro part 10322 is electrically connected to the lower surface 1033b of the conductive silicone strip 1033, the first electrical connection area 1023 of the transmitter 102 is electrically connected to the upper surface 1033d of the conductive silicone strip 1033 when the side surfaces 1033a and 1033c are not in use. An insulating material may be coated or adhered to the side surfaces 1033a and/or 1033c. Preferably, the side surfaces 1033a and 1033c are each coated or adhered to prevent the conductive areas on the two surfaces from being shorted by dirt.

In some embodiments of the invention, the insulating material may be one or more of rubber, silicone, polyethylene, fiberglass, epoxy, insulating lacquer, as long as good insulating properties are achieved.

In some embodiments of the invention, the thickness of the insulating material is 1-1000um, preferably the thickness of the insulating material is 10-100um.

In some embodiments of the invention, the insulating material may cover only the conductive areas of the conductive silicone strips 1033, and may also perform the function of insulating the surfaces of the non-electrically connected structures.

Referring to FIG. 7g, a top view of the conductive silicone strip 1033 is shown. In some embodiments of the invention, the first electrical connection area 1023 of the transmitter 102 is electrically connected to the upper surface 1033d of the conductive silicone strip 1033, in this case, the conductive areas not electrically connected to the first electrical connection area 1023 in the upper surface 1033d may be contaminated with dirt to cause a short circuit. In this case, the conductive areas not electrically connected to the first electrical connection area 1023 may be covered with an insulating material which is spaced apart on the upper surface 1033d. Alternatively, the in vitro part 10322 is electrically connected to the side surface 1033c of the conductive silicone strip 1033, and the conductive areas on the side surface 1033c that are not electrically connected to the in vitro part 10322 may be contaminated with dirt to cause short-circuiting, so that the conductive areas on the side surface 1033c that are not electrically connected to the in vitro part 10322 may be covered with an insulating material, which is spaced apart on the side surface 1033c.

Since the conductive areas and the insulating areas on the conductive silicone strip 1033 are spaced, the insulating material need only cover the conductive areas to perform its insulating function, thus in some embodiments of the invention, the insulating material may be continuously applied to the two adjacent conductive areas with an insulating area intermediate the two adjacent conductive areas, or, in other embodiments of the invention, the insulating material may be spaced apart from the two adjacent conductive areas without an insulating area intermediate the two adjacent conductive areas.

In some embodiments of the invention, the conductive silicone strip 1033 is covered with an insulating material on the surface of the structure where no electrical connection is made to other components, so that short-circuiting can be better avoided by dirt, and the detection signal stability can be improved.

In some embodiments of the invention, the in vitro part 10322 is curved or bent relative to the in vivo part 10321, and the in vivo part 10321 extends perpendicular or approximately perpendicular to and through the bottom case 101 to facilitate subcutaneous penetration of the user.

In some embodiments of the invention, the in vitro part 10322 is secured to the bottom case 101 by a sensor base, the sensor base is releasably coupled to the bottom case 101, the sensor base is detached from the bottom case 101 prior to installation, and the sensor base is assembled to the bottom case 101 during installation.

In other embodiments of the invention, the in vitro part 10322 lies directly on the bottom surface of the bottom case 101, eliminating the need for a sensor base structure, making the internal structure of the analyte detection device 10 more compact, which facilitates a miniaturized design.

In summary, the invention discloses a body fluid analyte detection device. The transmitter is releasably connected to the bottom case. After the user installs the disposable bottom case onto the skin surface, and then assembles the reusable transmitter onto the bottom case, a complete analyte detection device can be formed. When the user replaces the analyte detection device, they only need to apply force to one side of the bottom case to bend it and make the transmitter and the bottom case separate. Then, they can remove the bottom case from the skin to complete the disassembly. The bottom case is provided with a crease groove, which facilitates the bending of the bottom case. The structure is simple, the installation and disassembly operations are convenient, time-saving and labor-saving, and enhances the user's experience.

Although some specific embodiments of the invention have been described in detail by way of example, it should be understood by those skilled in the art that the above examples are for purposes of illustration only and are not intended to limit the scope of the invention. It will be appreciated by those skilled in the art that modifications may be made to the above embodiments without departing from the scope and spirit of the invention. The scope of the invention is defined by the appended claims.

## Claims

1. A body fluid analyte detection device, comprising:
a bottom case;
a transmitter, configured to establish a communication connection with a remote device;
a battery, configured to provide electrical energy for the body fluid analyte detection device;
a sensor and a conductive silicone strip installed on the bottom case; the sensor comprising an in vivo part and an in vitro part, the in vivo part is configured to penetrate the subcutaneous tissue to detect the parameters of the body fluid analyte information, when the transmitter is assembled onto the bottom case, the in vitro part is electrically connected to the transmitter through the conductive silicone strip to transmit the analyte parameters information; and
an adhesive tape, configured to stick the bottom case onto the skin surface;
wherein the bottom case is provided with a crease groove, the crease groove is thinner relative to the bottom surface of the bottom case, the crease groove comprises a straight section and a curved section, when separating the bottom case and the transmitter, the bottom case bends and fails along the crease groove.

2. The body fluid analyte detection device of claim 1, wherein the curved section is located at both ends of the straight section.

3. The body fluid analyte detection device of claim 1, wherein the curved section and/or the straight section are semi-open structures.

4. The body fluid analyte detection device of claim 1, wherein the conductive silicone strip is the rectangular structure.

5. The body fluid analyte detection device of claim 4, wherein the conductive silicone strip comprises conductive areas and insulating areas longitudinally spaced apart.

6. The body fluid analyte detection device of claim 5, wherein the transmitter comprises a first electrical connection area, the first electrical connection area and the in vitro part are respectively electrically connected to the conductive areas on the conductive silicone strip.

7. The body fluid analyte detection device of claim 6, wherein the first electrical connection area and the in vitro part are respectively electrically connected to the conductive areas on the adjacent or opposite surfaces of the conductive silicone strip.

8. The body fluid analyte detection device of claim 6, wherein at least one non-electrical connection structure surface of the conductive silicone strip is covered with insulating material.

9. The body fluid analyte detection device of claim 1, wherein the bottom case comprises at least one first engagement part, the transmitter comprises at least one second engagement part, when the transmitter is assembled onto the bottom case, the first engagement part is engaged with the second engagement part.

10. The body fluid analyte detection device of claim 9, wherein when the bottom case fails, the first engagement part decouples from the second engagement part.

11. The body fluid analyte detection device of claim 9, wherein the first engagement part is located on the curved side wall of the bottom case.

12. The body fluid analyte detection device of claim 1, wherein the bottom case comprises a battery cavity, the battery is set in the battery cavity.

13. The body fluid analyte detection device of claim 12, wherein the battery cavity comprises a cavity shell, the cavity shell is used as the shell of the battery.

14. The body fluid analyte detection device of claim 12, wherein the battery cavity comprises a separable cavity cover.

15. The body fluid analyte detection device of claim 1, wherein the device also comprises an auxiliary installer, which is configured to install the bottom case onto the skin surface and insert the in vivo part into the subcutaneous tissue.

16. The body fluid analyte detection device of claim 15, wherein before installation, the bottom case and the auxiliary installer are releasably connected, and the center axis of the bottom case and the auxiliary installer are coincident or not coincident.

17. The body fluid analyte detection device of claim 1, wherein the failure mode of the bottom case comprises bottom case fracture and bottom case bending deformation.

18. The body fluid analyte detection device of claim 1, wherein the in vitro part bends relative to the in vivo part.
